# EUROPÄISCHE PATENTSCHRIFT

(11) **EP 4 069 133 B1**
(45) Veröffentlichungstag und Bekanntmachung des Hinweises auf die Patenterteilung: **19.02.2025**
(21) Anmeldenummer: 20838328.1
(22) Anmeldetag: 04.12.2020
(51) Int. Cl.: A61C 7/10, A61B 17/66, A61C 7/00

(54) **DISTRAKTOR ZUR GAUMENERWEITERUNG UND VERFAHREN ZUR HERSTELLUNG DIESES DISTRAKTORS**
DISTRACTOR FOR PALATAL EXPANSION AND METHOD OF MANUFACTURING SAID DISTRACTOR
DISTRACTEUR POUR L'EXPANSION PALATINE ET PROCÉDÉ DE FABRICATION DUDIT DISTRACTEUR

(30) Priorität: 05.12.2019 AT 602682019; 27.02.2020 AT 600502020
(43) Veröffentlichungstag der Anmeldung: 12.10.2022
(73) Patentinhaber: Zellermayer, Elmar Gregor, 4600 Wels (AT)
(72) Erfinder: Zellermayer, Elmar Gregor, 4600 Wels (AT)
(74) Vertreter: Jell, Friedrich
(86) Internationale Anmeldenummer: PCT/AT2020/060435
(87) Internationale Veröffentlichungsnummer: WO 2021/108828

(56) Entgegenhaltungen:
- WO-A1-2016/034973
- WO-A1-2016/034973
- WO-A2-2008/011698
- WO-A2-2008/011698
- US-A1- 2015 056 566
- US-A1- 2015 056 566
- ORTHOLAB LABORATORIES: "Power Expander", 6 November 2019 (2019-11-06), Facebook, pages 1 - 1, XP055781119, Retrieved from the Internet <URL:https://www.facebook.com/LaboratoriosOrthoLab/photos/a.1402032640061521/2397922993805809/?type=3&theater> [retrieved on 20210302]
- MOON WON: "Class III treatment by combining facemask (FM) and maxillary skeletal expander (MSE)", SEMINARS IN ORTHODONTICS, vol. 24, no. 1, March 2018 (2018-03-01), US, pages 95 - 107, XP055780953, ISSN: 1073-8746, DOI: 10.1053/j.sodo.2018.01.009
- GRAF SIMON ET AL: "CAD-CAM design and 3-dimensional printing of mini-implant retained orthodontic appliances", AMERICAN JOURNAL OF ORTHODONTICS & DENTOFACIAL ORTHOPEDICS, vol. 154, no. 6, December 2018 (2018-12-01), pages 877 - 882, XP085547766, ISSN: 0889-5406, DOI: 10.1016/J.AJODO.2018.07.013
- ORTHOLAB LABORATORIES: "Power Expanders", 30 August 2019 (2019-08-30), Internet, pages 1 - 1, XP055782221, Retrieved from the Internet <URL:https://www.facebook.com/LaboratoriosOrthoLab/photos/a.1402032640061521/2343591289238980/?type=3&theater> [retrieved on 20210304]
- MOON WON: "Class III treatment by combining facemask (FM) and maxillary skeletal expander (MSE)", SEMINARS IN ORTHODONTICS, vol. 24, no. 1, March 2018 (2018-03-01), US, pages 95 - 107, XP055780953, ISSN: 1073-8746, DOI: 10.1053/j.sodo.2018.01.009
- GRAF SIMON ET AL: "CAD-CAM design and 3-dimensional printing of mini-implant retained orthodontic appliances", AMERICAN JOURNAL OF ORTHODONTICS & DENTOFACIAL ORTHOPEDICS, vol. 154, no. 6, December 2018 (2018-12-01), pages 877 - 882, XP085547766, ISSN: 0889-5406, DOI: 10.1016/J.AJODO.2018.07.013
- ORTHOLAB LABORATORIES: "Power Expanders", 30 August 2019 (2019-08-30), Internet, pages 1 - 1, XP055782221, Retrieved from the Internet <URL:https://www.facebook.com/LaboratoriosOrthoLab/photos/a.1402032640061521/2343591289238980/?type=3&theater> [retrieved on 20210304]

## Beschreibung

### Technisches Gebiet

Die Erfindung betrifft einen Distraktor zur Gaumenerweiterung, mit einem Expansionsteil, das mindestens zwei, im Abstand zueinander, entlang einer Längsachse einstellbare Expansionselemente aufweist, und mit zwei Verankerungsteilen, die je an einem Expansionselement befestigt sind, wobei jedes, durch die Längsachse in zwei Teilabschnitte geteilte Verankerungsteil sowohl an jedem der beiden Teilabschnitte mindestens eine Gaumenauflage, als auch mindestens eine Zahnabstützung aufweist, wobei jede Gaumenauflage eine Stützfläche zum Anliegen an den Gaumen und mindestens ein, im Bereich der Stützfläche vorgesehenes Durchgangsloch für ein Befestigungselement zur Verankerung mit einem Gaumenknochen des Gaumens aufweist, wobei jede Zahnabstützung eine erste Anlagefläche für eine, insbesondere palatinale, Seitenfläche eines Zahns und eine zweite Anlagefläche aufweist, die der ersten Anlagefläche vorkragt und eine, insbesondere okklusale, Zahnauflage für diesen Zahn ausbildet, um damit die Stützflächen am Gaumen zu positionieren.

### Stand der Technik

Verschiedenste Distraktoren zur Gaumenerweiterung sind aus dem Stand der Technik bekannt. Bei all diesen Distraktoren ist eine Verankerung im Mundraum eines Patienten vorgesehen, auf welche Verankerung ein Expansionsteil des Distraktors eine Spreizkraft ausübt, um damit den Gaumen zu verbreitern.

Beispielsweise offenbart die US 2016/0270884 A1 einen, sich an den Zähnen verankernden Distraktor. Nachteilig ist damit aber die Spreizkraft aufgrund des Halts der Zähne im Kiefer beschränkt, was dessen Anwendung auf sich im Wachstum befindliche Patienten einschränkt, deren Gaumennaht noch nicht vollständig verknöchert ist. Höhere Spreizkräfte auf den Gaumen - beispielsweise bei Erwachsenen - können mit, über Knochenschrauben am Gaumen verankerte Distraktoren erzeugt werden, wie diese beispielsweise aus der US 2009/0130620 A1 bekannt sind. Hierzu liegt der Distraktor mit einer Stützfläche am Gaumen an, welche Stützfläche über Knochenschrauben an den Gaumen angezogen ist. Diese Zugkräfte unterliegen nachteilig jedoch engen Grenzen, um einerseits für eine ausreichende Fixierung des Distraktors am Gaumenknochen zu sorgen und andererseits die Gaumenschleimhaut nicht zu überlasten, da dies zu erheblichen Entzündungsreaktionen im gesamten Mundraum führen kann. Zudem bedarf es bei diesen Distraktoren einer exakten axialen Positionierung gegenüber der Gaumennaht, um so eine über die Längserstreckung der Gaumennaht gleichmäßige Spreizkraft ausüben zu können.

Zur Erleichterung solch einer axialen Positionierung der Distraktoren ist beispielsweise aus der DE 10 2016 121 196 B3 ein Distraktor mit einer Kombination von Zahnund Gaumenverankerung bekannt. Ähnlich handhabbare Distraktoren werden als MARPE (Miniscrew Aided Rapid Palatal Expansion) oder MSE (Maxillary Skeletal Expander) etc. bezeichnet.

Ein weiterer Distraktor dieser Konstruktionsart ist als "Power Expander" von Herrn "Juan Carlos Perez Varela" bekannt (XP055782221). Dieser mit vier Knochenschrauben am Gaumen verankerte Distraktor weist eine Zahnabstützung an jedem, der beiden Verankerungsteile auf, welche Zahnabstützungen neben einer palatinalen Anlagefläche auch eine okklusale Zahnauflage ausbilden. Hierzu kragt die okklusale Zahnauflage der palatinalen Anlagefläche vor. Diese Zahnabstützung des "Power Expander" Distraktors erleichtert ebenso -wie von den anderen Distraktoren - bekannt, die Positionierung im Mundraum bzw. am Gaumen. Aber auch bei dieser "Power Expander" Lösung, besteht die Gefahr einer Überbelastung des Gaumens bzw. der Gaumenschleimhaut an der Stützfläche, was zu Entzündungsreaktionen im Bereich der Stützfläche führt und den Heilungsverlauf erheblich beeinträchtigen kann.

Ein weiterer Distrakror zur Gaumenerweiterung ist aus MOON WON: "Class III treatment by combining facemask (FM) and maxillary skeletal expander (MSE)", SEMINARS IN ORTHODONTICS, Bd. 24, Nr. 1, März 2018, Seiten 95-107 (XP055780953) bekannt.

### Darstellung der Erfindung

Die Erfindung hat sich daher ausgehend vom eingangs geschilderten Stand der Technik die Aufgabe gestellt, einen Distraktor zur Gaumenerweiterung dahingehend zu entwickeln, dass dieser trotz vergleichsweise hoher Spreizkräfte auf eine Gaumennaht sicher und unter verringerter Gefahr von Entzündungsreaktionen verwendet werden kann.

Die Erfindung löst die gestellte Aufgabe durch die Merkmale des Anspruchs 1.

Weisen die Verankerungsteile je mindestens zwei Zahnabstützungen auf, und wobei jeder Teilabschnitt mindestens eine der Zahnabstützungen und mindestens eine der Gaumenauflagen aufweist, mit der diese Zahnabstützung verbunden ist, können die Stützflächen in ihrer Position am Gaumen besonders vorteilhaft fixiert werden. Die beiden Zahnabstützungen stellen nämlich im Gegensatz zu anderen gaumenverankerten Distraktoren sicher, dass sich eine Stützfläche auch durch die Zugkräfte einer oder mehrerer Knochenschrauben nicht weiter in Richtung des Gaumens zu bewegen kann bzw. unerwünscht auf die Gaumenschleimhaut drücken kann. Damit können befestigungsbedingte Druckbelastungen bzw. Quetschungen an der Gaumenschleimhaut und damit einhergehende Entzündungsreaktionen im Bereich der Stützflächen ausgeschlossen werden. Der erfindungsgemäße Distraktor kann daher den gewünschten Heilungsverlauf reproduzierbar ermöglichen.

Zudem erleichtert diese Positionsfixierung auch die Verankerung des Distraktors, da beim Eindrehen der Knochenschrauben das Durchgangsloch der Gaumenauflage als Anschlag wirkt und so stets eine korrekte Befestigung am Gaumen sicherstellt. Der erfindungsgemäße Distraktor kann daher nicht nur den gewünschten Heilungsverlauf sicherstellen, sondern ist auch vergleichsweise handhabungsfreundlich zu verankern. Zudem steigert dies auch die Behandlungssicherheit - vor allem dann, wenn vergleichsweise hohe Spreizkräfte gefordert sind, beispielsweise, um den Gaumen eines, insbesondere erwachsenen, menschlichen Patienten zu erweitern.

Im Allgemeinen wird erwähnt, dass die Zahnabstützungen Teil einer Schiene sein können oder auch einzeln ausgebildet sein können. Im Allgemeinen ist weiter vorstellbar, dass beispielsweise eine, insbesondere direkte, Verbindung über einen Arm bzw. Steg oder mehrere Arme bzw. Stege ausgebildet ist. Als, insbesondere direkte, Verbindung ist auch eine Platte vorstellbar, in dem diese beispielsweise die beiden Zahnabstützungen mit der Gaumenauflage verbindet. Zudem wird erwähnt, dass der Begriff "insbesondere" als "beispielsweise" zu verstehen ist.

Die Positionsfixierung der Stützfläche kann selbst bei vergleichsweise hohen Anzugskräften der Knochenschrauben gewährleistet bleiben, wenn Gaumenauflagen und Zahnabstützungen des jeweiligen Verankerungsteils biegesteif miteinander verbunden sind - beispielsweise derart, dass keine Verformung beim Verankern des Distraktors am Gaumen eintritt.

Vorzugsweise sind beim jeweiligen Verankerungsteil die Gaumenauflagen und Zahnabstützungen biegesteif direkt miteinander verbunden. Diese direkte Verbindung kann beispielsweise über einen oder mehrere Arme ausgebildet sein. Auch ist als direkte Verbindung eine Platte vorstellbar.

Vorzugsweise ist die Verbindung zwischen Gaumenauflage und Zahnabstützung des jeweiligen Verankerungsteils berührungsfrei gegenüber dem Gaumen verlaufend ausgebildet, um unerwünschte Belastungen auf die Gaumenschleimhaut auszuschließen, was die Anwendungssicherheit des Distraktors weiter verbessern kann. Weiters kann dies einem verbesserten Tragekomfort des Distraktors dienlich sein.

Vorzugsweise weist der Verankerungsteil einen ersten und einen zweiten Arm auf, die jeweils eine Zahnabstützung mit einer Gaumenauflage verbinden. Damit kann der Distraktor einfach erfasst und/oder positioniert werden, was folglich die Handhabung des Distraktors weiter erleichtert.

Ist die Gaumenauflage als Öse ausgebildet, kann dies die Handhabung des Distraktors zusätzlich erleichtern bzw. die Konstruktion des Distraktors vereinfachen.

Weist der Verankerungsteil einen ersten und einen zweiten Arm auf, die jeweils eine Zahnabstützung mit einer Gaumenauflage verbinden, kann durch diese mechanische Kopplung die Positionsfixierung der Gaumenauflage am Gaumen weiter verbessert werden. Insbesondere, wenn Zahnabstützung und Gaumenauflage direkt verbunden werden. Zudem eröffnet sich damit die Möglichkeit, die Gaumenauflage in jeglicher Neigung an die Zahnabstützung anzubinden, um so unterschiedlichen Gaumenkonturen folgen zu können. Dies kann unter anderem zu einer verbesserten Verankerung mit dem Gaumenknochen beitragen.

Letzteres insbesondere dann, wenn die Gaumenauflage am ersten oder zweiten Arm direkt anschließt. Zudem kann dies zu einer besonders steifen mechanischen Verbindung zwischen Zahnabstützung und Gaumenauflage führen, was die Standfestigkeit der Positionsfixierung verbessern kann.

Die Handhabung des Distraktors kann weiter erleichtert werden, wenn die Zahnabstützung einen dritten Arm aufweist, der die Gaumenauflagen direkt verbindet und der mit dem jeweiligen Expansionselement fest verbunden ist. Zudem ist die Konstruktion des Distraktors somit vereinfachbar. Zudem kann damit die Anpassbarkeit der Gaumenauflage auf verschiedenste Neigungen gesteigert werden, um so unterschiedlichen Gaumenkonturen folgen zu können.

Alternativ zu mehreren Armen kann die Verbindung auch einen Steg aufweisen. Hierzu sind bei jedem Verankerungsteil die Zahnabstützungen über einen gemeinsamen Steg mit den Gaumenauflagen verbunden. Damit ist diesen beiden Teilabschnitten des jeweiligen Verankerungsteils auch dieser Steg gemeinsam. Unter anderem kann damit der Materialaufwand am Verankerungsteil verringert werden.

Teilt sich der Steg in zwei Schenkel auf, die an je eine Gaumenauflage anschließen, kann die Konstruktion des Verankerungsteil weiter vereinfacht werden. Zudem kann damit ein schlankes Verankerungsteil für eine minimierte Gaumenüberdeckung geschaffen werden, was beispielsweise den Tragkomfort verbessern kann.

Verlaufen die Schenkel zueinander v-förmig kann dies die schlanke Bauform des Verankerungsteil weiter begünstigen.

Dies umso mehr, wenn das jeweilige Expansionselement an den Steg anschließt, der sich in diesem Bereich in die beiden Schenkel aufteilt.

Vorzugsweise weist jedes Verankerungsteil zwei Gaumenauflagen und/oder zwei, drei oder vier Zahnabstützungen auf, um damit den Distraktor spannungsfrei positionieren und auch verankern zu können.

Die Konstruktion kann weiter vereinfacht werden, wenn die erste Zahnabstützung ein molares Band aufweist, das die erste Anlagefläche ausbildet.

Die Handhabung des Distraktors kann sich weiter verbessern, wenn die zweite Zahnabstützung ein halbes Lingualband aufweist, das die erste Anlagefläche ausbildet.

Sind die Verankerungsteile einteilig, insbesondere durch ein additives Fertigungsverfahren einteilig, ausgebildet, stellt dies durch eine vergleichsweise hohe Formgenauigkeit eine exakte Positionierung und Fixierung des Distraktors am Gaumen sicher.

Die mechanische Belastbarkeit des Distraktors lässt sich weiter verbessern, wenn der Verankerungsteil aus einer Kobalt-Chrom-Wolfram-Legierung, insbesondere Co63,9Cr24,7W5,5Mo5.0Si, besteht.

Die Fixierung der Stützflächen in ihrer Position am Gaumen kann verbessert werden, wenn zur Längsachse am weitesten außen liegenden Zahnabstützungen der beiden Verankerungsteile zwischen sich eine Abstützfläche aufspannen, innerhalb der die Gaumenauflagen angeordnet sind. Beispielsweise kann dadurch beim Fixieren des Distraktors mit Gaumenschrauben ein Verkippen standfest verhindert werden. Die Gefahr einer Überbelastung des Gaumens bzw. der Gaumenschleimhaut an der Stützfläche kann so nicht eintreten und den Heilungsverlauf nicht beeinträchtigen.

Zur Fixierung der Position der Stützflächen am Gaumen kann ausreichend sein, wenn die erste Zahnabstützung eines jeden Verankerungsteils an einem hinteren Backenzahn vorsehbar ausgebildet ist und/oder die zweite Zahnabstützung eines jeden Verankerungsteils an einem vorderen Backenzahn vorsehbar ausgebildet ist.

Zur Fixierung der Position der Stützflächen am Gaumen kann ausreichend sein, wenn die erste Zahnabstützung eines jeden Verankerungsteils an einem ersten Zahn, der ein hinterer Backenzahn ist, vorsehbar ausgebildet ist und/oder die zweite Zahnabstützung eines jeden Verankerungsteils an einem zweiten Zahn, der ein vorderer Backenzahn ist, vorsehbar ausgebildet ist.

Vorzugweise sind die Stützflächen der Gaumenauflagen jeweils der Kontur des Gaumens, insbesondere der Gaumenschleimhaut, folgend ausgebildet, was den gewünschten Behandlungsverlauf weiter verbessern kann.

Vorzugsweise sind die Zahnabstützungen jeweils der Kontur des betreffenden Zahns folgend ausgebildet, was den Tragekomfort des Distraktors weiter verbessern kann.

Die Erfindung hat sich außerdem die Aufgabe gestellt, ein Verfahren zu schaffen, mit dem der erfindungsgemäße Distraktor formgenau hergestellt werden kann.

Die Erfindung löst die gestellte Aufgabe durch die Merkmale des Anspruchs 21.

Wird ein digitales Abbild zumindest eines Abschnitts eines Kiefers eines Patienten erfasst, die Stützflächen und Zahnabstützungen an das digitale Abbild angepasst und mithilfe eines additiven Fertigungsverfahrens die Verankerungsteile mit den angepassten Stützflächen und Zahnabstützungen hergestellt, ist eine besonders hohe Passgenauigkeit des Distraktors zum jeweiligen Kiefer bzw. Gaumens des jeweiligen Patienten möglich. Dies kann insbesondere den gewünschten Behandlungsverlauf sicherstellen.

### Kurzbeschreibung der Zeichnungen

In den Figuren ist beispielsweise der Erfindungsgegenstand anhand mehrere Ausführungsvarianten näher dargestellt. Es zeigen
- Fig. 1: eine Draufsicht auf einen ersten Distraktor, positioniert an einem Gaumen, nach einer ersten Ausführungsvariante,
- Fig. 2: eine Unteransicht auf den nach Fig. 1 dargestellten Distraktor und
- Fig. 3: eine nach III-III geschnittene Seitenansicht des Distraktors der Fig. 1 und
- Fig. 4: eine Draufsicht auf einen zweiten Distraktor, positioniert an einem Gaumen, nach einer zweiten Ausführungsvariante und
- Fig. 5: eine vergrößerte Draufsicht auf den Distraktor nach Fig. 4.

### Wege zur Ausführung der Erfindung

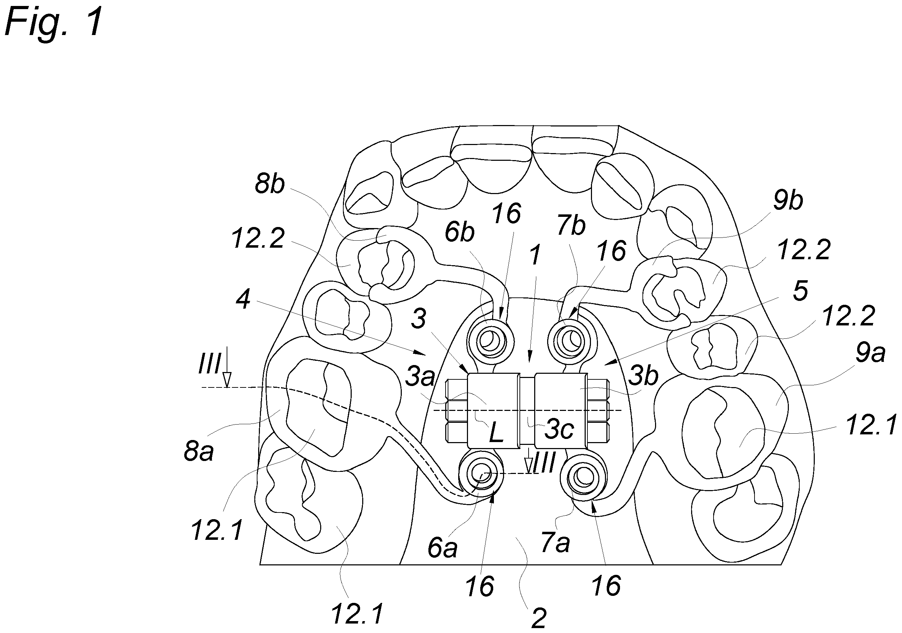

Nach Fig. 1 ist der erfindungsgemäße Distraktor 1 nach einer ersten Ausführungsvariante ohne Befestigungselemente dargestellt, der an eine Nachbildung eines Gaumens 2 samt Zahnbogen positioniert ist.

Der Distraktor 1 weist ein Expansionsteil 3 mit zwei, im Abstand zueinander einstellbaren Expansionselemente 3a, 3b auf. Diese Einstellung entlang einer Längsachse L des Expansionsteils 3 erfolgt über einen Spindeltrieb 3c, der an beide Expansionselemente 3a, 3b angreift.

Des Weiteren weist der Distraktor 1 zwei Verankerungsteile 4, 5 mit mehreren Gaumenauflagen 6a, 6b, 7a, 7b und mehreren Zahnabstützungen 8a, 8b bzw. 9a, 9b auf.

Die Verankerungsteile 4, 5 sind mit je einem Expansionselement 3a, 3b des Distraktors 1 fest verbunden bzw. daran befestigt.

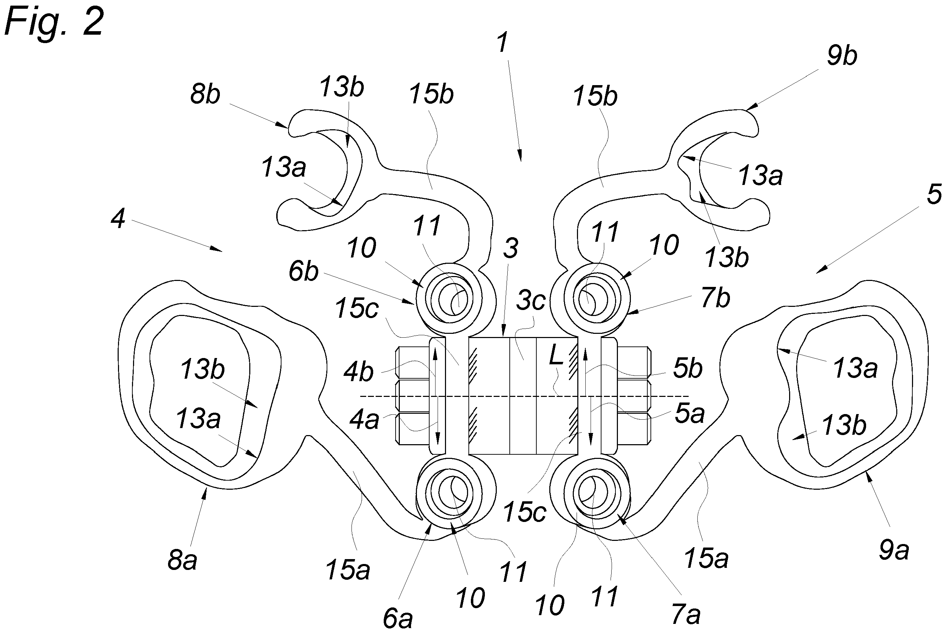

Die mehreren, im Ausführungsbeispiel vier, Gaumenauflagen 6a, 6b, 7a, 7b dienen der Verankerung des Distraktors 1 am Gaumen 2. An jedem Verankerungsteil 4 bzw. 5 sind zwei Gaumenauflagen 6a, 6b bzw. 7a, 7b vorgesehen, und zwar je eine Gaumenauflage 6a, 6b, 7a bzw. 7b an einem Teilabschnitt 4a, 4b bzw. 5a, 5b des jeweiligen Verankerungsteils 4 bzw. 5, wobei sich die zwei Teilabschnitte 4a, 4b bzw. 5a, 5b durch eine Teilung des jeweiligen Verankerungsteil 4 bzw. 5 an der Längsachse L des Expansionsteils 3 ergeben, was nach Fig. 2 näher dargestellt ist.

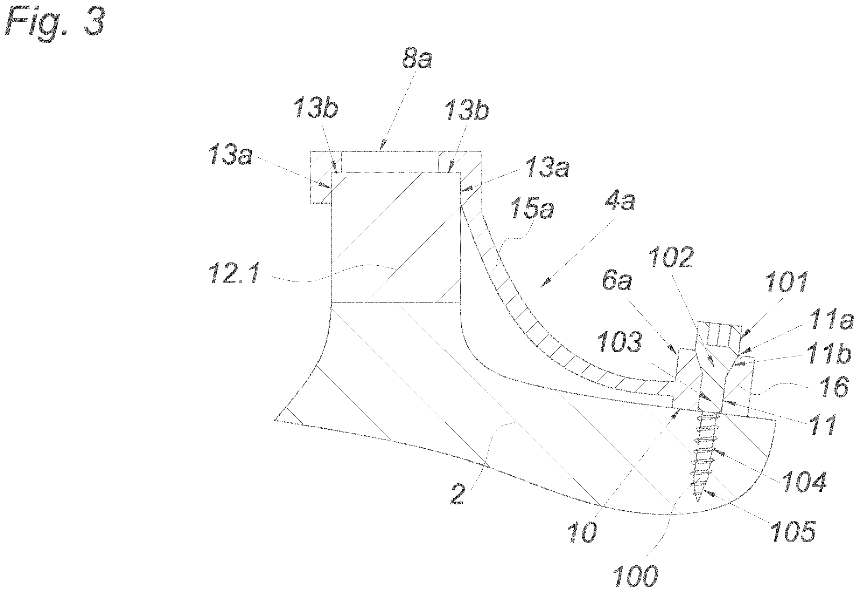

Die Gaumenauflagen 6a, 6b, 7a, 7b weisen des Weiteren je eine Stützfläche 10 auf, über welche der Distraktor 1 am Gaumen 2 bzw. an der Gaumenschleimhaut anliegt. Diese kreisringförmigen Stützflächen 10 sind in der Fig. 2 erkennbar. Im Bereich der Stützflächen 10 der Gaumenauflagen 6a, 6b, 7a, 7b ist zudem je ein Durchgangsloch 11 vorgesehen, über das -wie beispielsweise nach Fig. 3 dargestellt- vier Knochenschrauben 100 als Befestigungselemente den Distraktor 1 am Gaumen 2 verankern. Damit diese Verankerung positionsgenau am Gaumen 2 stattfindet, weist der Distraktors 1 mehrere Zahnabstützungen 8a, 8b bzw. 9a, 9b auf.

Davon weist jede Zahnabstützung 8a, 8b, 9a, 9b eine erste Anlagefläche 13a für eine palatinale Seitenfläche eines ersten Zahns 12.1, der ein hinterer Backenzahn (Molar) ist, und eine zweite Anlagefläche 13b auf, die der ersten Anlagefläche 13a vorkragt und eine okklusale Zahnauflage für diesen Zahn 12.1 ausbildet -siehe hierzu insbesondere Figur 3. Anhand dieser Zahnabstützungen 8a, 8b, 9a, 9b kann nun die Stützfläche 10 am Gaumen 2 positioniert - und damit der Distraktor 1 am Gaumen 2 ausgerichtet werden.

Erfindungsgemäß wird dieses Anliegen der Stützflächen 10 am Gaumen 2 in der Position auch fixiert, indem die Verankerungsteile 4, 5 je mindestens zwei Zahnabstützungen 8a, 8b, 9a, 9b für je einen Zahn 12.1, 12.2 aufweisen, und wobei bei jedem Teilabschnitt 4a, 4b bzw. 5a, 5b mindestens eine Zahnabstützung 8a, 8b, 9a, 9b mit jeweils einer Gaumenauflage 6a, 6b, 7a, 7b verbunden ist - siehe hierzu insbesondere Figuren 1, 2 bzw. 4, 5.

Beide Teilabschnitte 4a, 4b bzw. 5a, 5b des jeweiligen Verankerungsteils 4 bzw. 5 weisen daher je eine Zahnabstützung 8a, 8b, 9a, 9b und eine Gaumenauflage 6a, 6b, 7a, 7b auf und werden sohin samt deren Gaumenauflagen 6a, 6b bzw. 7a, 7b in der Position fixiert bzw. werden damit die Stützflächen 10 in ihrer Position am Gaumen 2 fixiert .

Nach Fig. 1 sind von den Zahnabstützungen 8a, 8b, 9a, 9b einerseits die ersten Zahnabstützungen 8a, 9a an den ersten Zahn 12.1, der ein hinterer Backenzahn (Molar) ist, und andererseits die zweiten Zahnabstützungen 8b, 9b an den zweiten Zahn 12.2, der ein vorderer Backenzahn (Premolar) ist, vorsehbar ausgebildet.

Ein angezogenes Befestigungselement kann daher die Stützflächen 10 nicht in Richtung Gaumen 2 ziehen - eine Belastung der Gaumenschleimhaut wird so stets vermieden. Demnach ist die Gefahr der Entstehung von Entzündungsreaktionen an der Gaumenschleimhaut im Bereich der Stützflächen 10, welche den Heilungsverlauf erheblich nachteilig beeinflussen kann, nicht gegeben.

Dies insbesondere, weil die Stützflächen 6a, 6b, 7a, 7b der Gaumenauflagen jeweils der Kontur der Gaumenschleimhaut des Gaumens 2 folgend ausgebildet sind, und zwar dort wo diese jeweils an den Gaumen 2 anliegen. Der Tragekomfort des Distraktors 2 ist besonders hoch, da die Zahnabstützungen 8a, 8b, 9a, 9b jeweils der Kontur des betreffenden Zahns 12.1, 12.2 folgend ausgebildet sind.

Auch eröffnet sich durch diese erfindungsgemäße konstruktive Ausführung die Möglichkeit, die Durchgangslöcher 11 der Gaumenauflagen 6a, 6b, 7a bzw. 7b relativ zueinander auf besondere Weise zu positionieren. Beispielsweise sind diese Durchgangslöcher 11 innerhalb einer Molarbox (bzw. im Gaumenabschnitt zwischen den sich gegenüberliegenden Molarzähnen) und jeweils im gleichen Abstand zur Sutura palatina mediana des Oberkiefers am Distraktor 1 vorsehbar. Dies garantiert einen sicheren Behandlungserfolg.

Wie nach Fig. 1 zu erkennen, sind Gaumenauflage 6a, 6b, 7a, 7b und Zahnabstützung 8a, 8b bzw. 9a, 9b über einen ersten bzw. zweiten Arm 15a bzw. 15b biegesteif miteinander direkt verbunden. Dadurch können auch die Gaumenauflagen 6a, 6b, 7a, 7b auch mit unterschiedlichen Neigungswinkeln zum Gaumen 2 vorgesehen werden und sind damit besonders an den Verlauf des Gaumens 2 anpassungsfähig. Insbesondere können diese in der Neigung auch so eingestellt werden, dass die Befestigungselemente eine erhöhte Überlappung mit dem Gaumenknochen erfahren und damit die Fixierung des Distraktors 1 verbessern.

Konstruktiv einfach ausgeführt, schließen die Gaumenauflagen 6a, 6b, 7a, 7b in der Form einer Öse 16 an den Arm 15a, 15b direkt an. An die jeweiligen Gaumenauflagen 6a, 6b, 7a, 7b des Verankerungsteils 4 bzw. 5 schließt ein dritter Arm 15c an - wie in Fig. 2 zu erkennen ist.

Dieser dritte Arm 15c ist mit dem jeweiligen Expansionselement 3a, 3b fest - und zwar stoffschlüssig über nach Fig. 2 schematisch dargestellte Schweißverbindungen - verbunden. Damit können die Verankerungsteile 4 bzw. 5 unabhängig vom Expansionsteil 3, beispielsweise durch ein additives Fertigungsverfahren, einteilig ausgebildet werden. Eine exakte Anpassung der Verankerungsteile 4 bzw. 5 an den Gaumen 2 ist dadurch möglich. Vorzugsweise sind zumindest die Stützflächen 10 oder die Verankerungsteile 4 bzw. 5 elektropoliert. Vorzugsweise werden die Verankerungsteile 4, 5 aus einer Kobalt-Chrom-Wolfram-Legierung gefertigt - vorzugsweise aus Co63,9Cr24,7W5,5Mo5.0Si.

Außerdem ist in der Fig. 2 zu erkennen, dass die erste Zahnabstützung 8a, 9a eines Verankerungsteils 4 bzw. 5 ein molares Band aufweist, das die erste Anlagefläche 13a ausbildet. An das molare Band schließt die okklusale Zahnauflage an. Die zweite Zahnabstützung 8b bzw. 9b eines Verankerungsteils 4 bzw. 5 weist ein halbes Lingualband auf, das die erste Anlagefläche 13a ausbildet. An das halbe Lingualband schließt die okklusale Zahnauflage 13b an.

Für eine handhabungsfreundliche Verankerung des Distraktors 1 sorgen die vier Knochenschrauben 100. Die jeweilige Knochenschraube 100 durchgreift das Durchgangsloch 11 der jeweiligen Zahnabstützung 8a, 8b, 9a, 9b, wobei das Durchgangsloch 11 einen sich vom Lochrand 11a aus konisch verjüngenden Endabschnitt 11b aufweist, wie beispielsweise in der Fig. 3 für die Zahnabstützung 8a dargestellt.

Dieser Endabschnitt 11b dient als Anschlag für die Knochenschraube 100, nämlich als Anschlag für einen kegelstumpfförmigen Kopfabschnitt 102 am Schraubenkopf 101 der Knochenschraube 100. Ein Überdrehen der Knochenschraube 100 kann daher nicht stattfinden. Vorzugsweise werden die Zahnabstützungen 8a, 8b, 9a, 9b mit dem jeweiligen Zahn verklebt, und danach der Distraktor 1 mit den Knochenschrauben 100 am Gaumen 2 verankert.

Die Knochenschraube 100 weist zudem am Schaft 103 ein Außengewinde 104 und am Schaftende eine Abflachung 105 auf. Zudem weist die Knochenschraube 100 am Kopf einen Innenkonus auf, über den die Knochenschraube 100 handhabungsfreundlich betätigt werden kann.

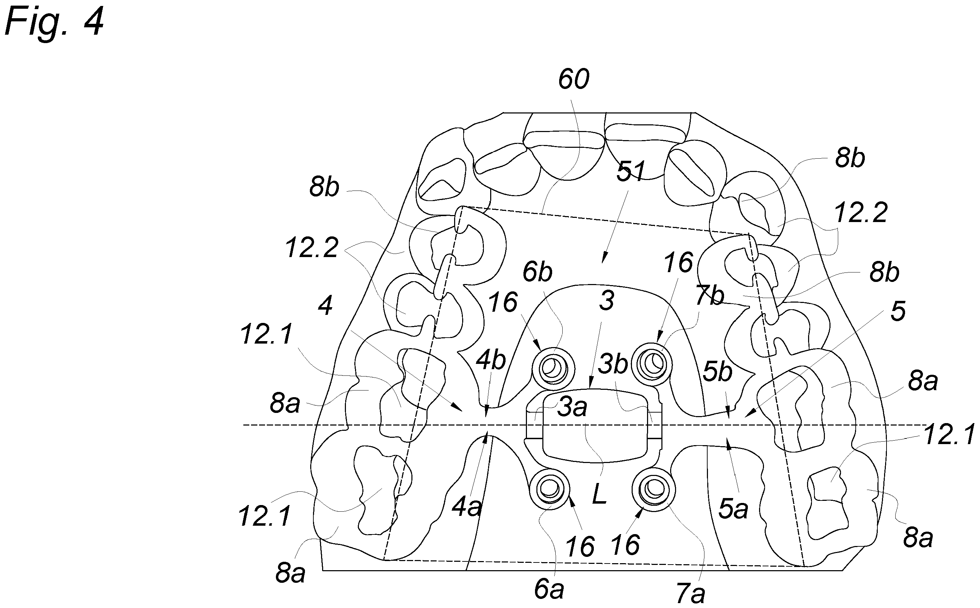

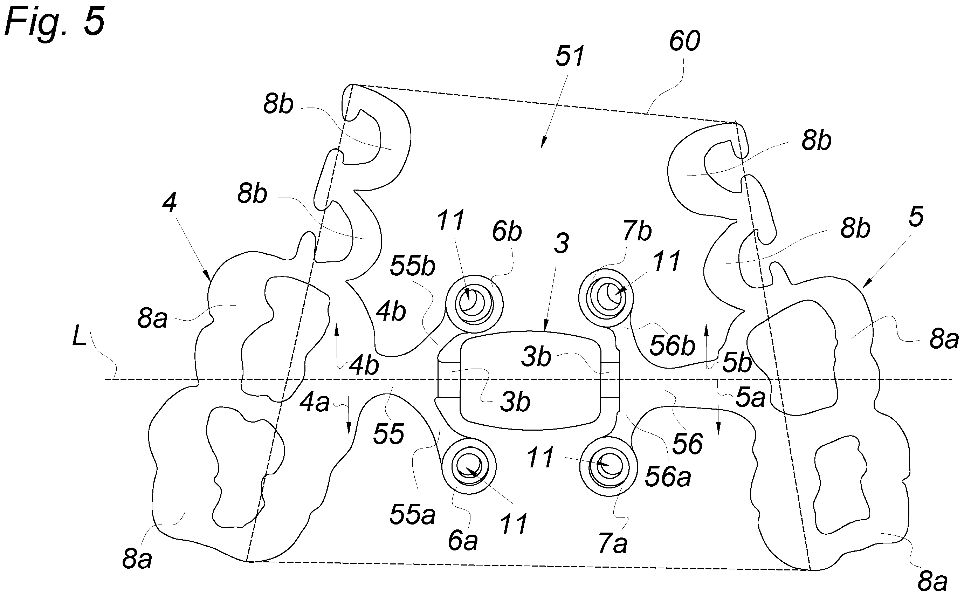

Nach Fig. 4 und Fig. 5 wird ein weiterer erfindungsgemäßer Distraktor 51 nach einer zweiten Ausführungsvariante ohne Befestigungselemente in Draufsicht dargestellt.

Zum Unterschied zum Distraktor 1 der Figuren 1 bis 2, weisen die Verankerungsteile des Distraktors 51 anders konstruierte Verankerungsteile 4, 5 auf. So ist deren Verbindung zwischen den jeweiligen Zahnabstützungen 8a, 8b bzw. 9a, 9b und den Gaumenauflagen 6a, 6b bzw. 7a, 7b verschieden.

Bei jedem Verankerungsteil 4, 5 ist hier ein gemeinsamer Steg 55 bzw. 56 vorgesehen, der von den Zahnabstützungen 8a, 8b bzw. 9a, 9b ausgeht, an das jeweiligen Expansionselement 3a, 3b anschließt und sich dort in zwei Schenkel 55a, 55b bzw. 56a, 56b aufteilt, die an je eine Gaumenauflage 6a, 6b bzw. 7a, 7b anschließen, wie insbesondere in Fig. 5 zu erkennen.

Diese Verbindungen zwischen den jeweiligen Zahnabstützungen 8a, 8b bzw. 9a, 9b und den Gaumenauflagen 6a, 6b bzw. 7a, 7b sind Y-förmig ausgeführt, was zu besonders kompakten Verankerungsteilen 4, 5 führt. Dies erhöht unter anderem den Tragekomfort des Distraktors 51.

Außerdem weist der Distraktor 51 vier Zahnabstützungen 8a, 8b bzw. 9a, 9b pro Verankerungsteil 4, 5 auf, was zu einer besonders breiten Abstützfläche 60 führt. Diese Abstützfläche 60 wird zwischen von den zur Längsachse L am weitesten außen liegenden Zahnabstützungen 8a, 8b, 9a, 9b der beiden Verankerungsteile 4, 5 aufgespannt. Innerhalb der Abstützfläche 60 sind die Gaumenauflagen 6a, 6b, 7a, 7b angeordnet, was für eine hohe Stabilität in der Positionsfixierung des Distraktors bzw. der Gaumenauflagen 6a, 6b, 7a, 7b gegenüber dem Gaumen führt.

Aber auch bei diesem Distraktor 51 weisen die Verankerungsteile 4, 5 je mindestens zwei, nämlich vier, Zahnabstützungen 8a, 8b, 9a, 9b auf. Vergleichbar mit dem Distraktor 1 weist hier auch jeder Teilabschnitt 4a, 4b bzw. 5a, 5b der Verankerungsteile 4, 5 mindestens eine der Zahnabstützungen 8a, 8b bzw. 9a, 9b und mindestens eine der Gaumenauflagen 6a, 6b bzw. 7a, 7b auf. In diesem Fall weist der erste Teilabschnitt 4a, 5a eine erste Zahnabstützung 8a an einem ersten Zahn 12.1 und der zweite Teilabschnitt 4b, 5b zwei zweite Zahnabstützungen 8b je an einem zweiten Zahn 12.2 auf. Eine erste Zahnabstützung 8a an einem Zahn 12.1 ist diesen beiden Teilabschnitten 4a, 5b gemeinsam.

Vorstellbar sind auch drei Zahnabstützungen 8a, 8b bzw. 9a, 9b pro Verankerungsteil 4, 5, was nicht näher dargestellt worden ist.

Zur Herstellung eines Distraktors 1, 51 wird vorteilhaft ein digitales Abbild des Kiefers eines Patienten erfasst. Die Stützflächen 10 und Zahnabstützungen 8a, 8b, 9a, 9b werden an das digitale Abbild des Kiefers angepasst, wodurch der Distraktors 1, 51 eine hohe Passgenauigkeit sicherstellen kann. Vorzugsweise werden mithilfe eines additiven Fertigungsverfahrens die Verankerungsteile 4, 5 mit den angepassten Stützflächen 10 und Zahnabstützungen 8a, 8b, 9a, 9b hergestellt, was das Herstellverfahren vereinfacht und trotz hoher Individualität aufgrund unterschiedlicher Kieferformen die Herstellung des Distraktors 1, 51 beschleunigt.

## Patentansprüche

1. Distraktor zur Gaumenerweiterung, mit einem Expansionsteil (3), das mindestens zwei, im Abstand zueinander, entlang einer Längsachse (L) einstellbare Expansionselemente (3a, 3b) aufweist, und mit zwei Verankerungsteilen (4, 5), die je an einem Expansionselement (3a, 3b) befestigt sind, wobei jedes, durch die Längsachse (L) in zwei Teilabschnitte (4a, 4b bzw. 5a, 5b) geteilte Verankerungsteil (4, 5) sowohl an jedem der beiden Teilabschnitte (4a, 4b bzw. 5a, 5b) mindestens eine Gaumenauflage (6a, 6b, 7a, 7b), als auch mindestens eine Zahnabstützung (8a, 8b, 9a, 9b) aufweist, wobei jede Gaumenauflage (6a, 6b, 7a, 7b) eine Stützfläche (10) zum Anliegen an den Gaumen (2) und mindestens ein, im Bereich der Stützfläche (10) vorgesehenes Durchgangsloch (11) für ein Befestigungselement zur Verankerung mit einem Gaumenknochen des Gaumens (2) aufweist, wobei jede Zahnabstützung (8a, 8b, 9a, 9b) eine erste Anlagefläche (13a)für eine palatinale Seitenfläche eines Zahns (12.1, 12.2) und eine zweite Anlagefläche (13b) aufweist, die der ersten Anlagefläche (13a) vorkragt und eine okklusale Zahnauflage für diesen Zahn (12.1, 12.2) ausbildet, um damit die Stützflächen (10) am Gaumen (2) zu positionieren, wobei die Verankerungsteile (4, 5) je mindestens zwei Zahnabstützungen (8a, 8b, 9a, 9b) aufweisen, und wobei jeder Teilabschnitt (4a, 4b bzw. 5a, 5b) mindestens eine der Zahnabstützungen (8a, 8b bzw. 9a, 9b) und mindestens eine der Gaumenauflagen (6a, 6b bzw. 7a, 7b) aufweist, mit der diese Zahnabstützung (8a, 8b bzw. 9a, 9b) verbunden ist, um damit die Stützflächen (10) in ihrer Position am Gaumen (2) zu fixieren.

2. Distraktor nach Anspruch 1, **dadurch gekennzeichnet, dass** Gaumenauflagen (6a, 6b, 7a, 7b) und Zahnabstützungen (8a, 8b, 9a, 9b) des jeweiligen Verankerungsteils (4, 5), insbesondere biegesteif, direkt miteinander verbunden sind.

3. Distraktor nach Anspruch 1 oder 2, **dadurch gekennzeichnet, dass** die Verbindung zwischen Gaumenauflagen (6a, 6b, 7a, 7b) und Zahnabstützungen (8a, 8b, 9a, 9b) des jeweiligen Verankerungsteils (4, 5) berührungsfrei gegenüber dem Gaumen (2) verlaufend ausgebildet ist.

4. Distraktor nach einem der Ansprüche 1 bis 3, **dadurch gekennzeichnet, dass** die Gaumenauflage (6a, 6b, 7a, 7b) als Öse (16) ausgebildet ist.

5. Distraktor nach einem der Ansprüche 1 bis 4, **dadurch gekennzeichnet, dass** das Verankerungsteil (4, 5) einen ersten und einen zweiten Arm (15a, 15b) aufweist, die jeweils eine Zahnabstützung (8a, 8b, 9a, 9b) mit einer Gaumenauflage (6a, 6b, 7a, 7b), insbesondere direkt, verbinden.

6. Distraktor nach Anspruch 4 und 5, **dadurch gekennzeichnet, dass** die Gaumenauflage (6a, 6b, 7a, 7b) am ersten oder zweiten Arm (15a, 15b, 15c) direkt anschließt.

7. Distraktor nach Anspruch 5 oder 6, **dadurch gekennzeichnet, dass** die Zahnabstützung (8a, 8b, 9a, 9b) einen dritten Arm (15c) aufweist, der die Gaumenauflagen (6a, 6b, 7a, 7b) direkt verbindet und der mit dem jeweiligen Expansionselement (3a, 3b) fest verbunden ist.

8. Distraktor nach einem der Ansprüche 1 bis 4, **dadurch gekennzeichnet, dass** bei jedem Verankerungsteil (4, 5) die Zahnabstützungen (8a, 8b bzw. 9a, 9b) über einen gemeinsamen Steg (55, 56) mit den Gaumenauflagen (6a, 6b bzw. 7a, 7b) verbunden sind.

9. Distraktor nach Anspruch 8, **dadurch gekennzeichnet, dass** sich der Steg (55, 56) in zwei Schenkel (55a, 55b bzw. 56a, 56b) aufteilt, die an je eine Gaumenauflage (6a, 6b bzw. 7a, 7b) anschließen.

10. Distraktor nach Anspruch 8 oder 9, **dadurch gekennzeichnet, dass** die Schenkel (55a, 55b bzw. 56a, 56b) zueinander v-förmig verlaufen und/oder dass das jeweilige Expansionselement (3a, 3b) an den Steg (55, 56) anschließt, der sich in diesem Bereich in die beiden Schenkel (55a, 55b bzw. 56a, 56b) aufteilt.

11. Distraktor nach einem der Ansprüche 1 bis 10, **dadurch gekennzeichnet, dass** jedes Verankerungsteil (4, 5) zwei Gaumenauflagen (6a, 6b, 7a, 7b) und/oder zwei, drei oder vier Zahnabstützungen (8a, 8b, 9a, 9b) aufweist.

12. Distraktor nach einem der Ansprüche 1 bis 11, **dadurch gekennzeichnet, dass** die erste Zahnabstützung (8a, 9a) ein molares Band aufweist, das die erste Anlagefläche (13a) ausbildet.

13. Distraktor nach einem der Ansprüche 1 bis 12, **dadurch gekennzeichnet, dass** die zweite Zahnabstützung (8b, 9b) ein halbes Lingualband aufweist, das die erste Anlagefläche (13a) ausbildet.

14. Distraktor nach einem der Ansprüche 1 bis 13, **dadurch gekennzeichnet, dass** die Verankerungsteile (4, 5) einteilig, insbesondere durch ein additives Fertigungsverfahren einteilig, ausgebildet sind.

15. Distraktor nach einem der Ansprüche 1 bis 14, **dadurch gekennzeichnet, dass** das Durchgangsloch (11) einen sich vom Lochrand (11a) aus konisch verjüngenden Endabschnitt (11b) aufweist.

16. Distraktor nach einem der Ansprüche 1 bis 15, **dadurch gekennzeichnet, dass** die Verankerungsteile (4, 5) aus einer Kobalt-Chrom-Wolfram-Legierung, insbesondere Co63,9Cr24,7W5,5Mo5.0Si, bestehen.

17. Distraktor nach einem der Ansprüche 1 bis 16, **dadurch gekennzeichnet, dass** die zur Längsachse am weitesten außen liegenden Zahnabstützungen (8a, 8b, 9a, 9b) der beiden Verankerungsteile (4, 5) zwischen sich eine Abstützfläche (60) aufspannen, innerhalb der die Gaumenauflagen (6a, 6b, 7a, 7b) angeordnet sind.

18. Distraktor nach einem der Ansprüche 1 bis 17, **dadurch gekennzeichnet, dass** die erste Zahnabstützung (8a, 9a) eines jeden Verankerungsteils (4, 5) an einem ersten Zahn (12.1), der ein hinterer Backenzahn ist, vorsehbar ausgebildet ist und/oder die zweite Zahnabstützung (8b, 9b) eines jeden Verankerungsteils (4, 5) an einem zweiten Zahn (12.2), der ein vorderer Backenzahn ist, vorsehbar ausgebildet ist.

19. Distraktor nach einem der Ansprüche 1 bis 18, **dadurch gekennzeichnet, dass** die Stützflächen (10) der Gaumenauflagen (6a, 6b, 7a, 7b) jeweils der Kontur des Gaumens (2) folgend und/oder die Zahnabstützungen (8a, 8b, 9a, 9b) jeweils der Kontur des betreffenden Zahns (12.1, 12.2) folgend ausgebildet sind.

20. Verfahren zur Herstellung eines Distraktors (1, 51) nach einem der Ansprüche 1 bis 19, **dadurch gekennzeichnet, dass** ein digitales Abbild zumindest eines Abschnitts eines Kiefers eines Patienten erfasst wird, die Stützflächen (10) und Zahnabstützungen (8a, 8b, 9a, 9b) an das digitale Abbild angepasst und mithilfe eines additiven Fertigungsverfahrens die Verankerungsteile (4, 5) mit den angepassten Stützflächen (10) und Zahnabstützungen (8a, 8b, 9a, 9b) hergestellt werden.

## Claims

1. Distractor for palatal expansion, with an expansion part (3) that has at least two expansion elements (3a, 3b) spaced apart from each other that are adjustable along a longitudinal axis (L) and with two anchoring parts (4, 5) that are each fastened to a respective expansion element (3a, 3b), wherein each anchoring part (4, 5) that is divided into two subsections (4a, 4b and 5a, 5b, respectively) by the longitudinal axis (L) has at least one palate rest (6a, 6b, 7a, 7b) and at least one tooth support (8a, 8b, 9a, 9b) in each of the two subsections (4a, 4b and 5a, 5b, respectively), wherein each palate rest (6a, 6b, 7a, 7b) has a support surface (10) for contacting the palate (2) and at least one through hole (11) provided in the region of the support surface (10) to accommodate a fastening element for anchoring to a palatine bone of the palate (2), wherein each tooth support (8a, 8b, 9a, 9b) has a first contact surface (13a) for an palatal side surface of a tooth (12.1, 12.2) and a second contact surface (13b), which overhangs the first contact surface (13a) and forms an occlusal tooth rest for this tooth (12.1, 12.2), in order to thus position the support surfaces (10) against the palate (2), wherein the anchoring parts (4, 5) each have at least two tooth supports (8a, 8b, 9a, 9b) and wherein each subsection (4a, 4b and 5a, 5b, respectively) has at least one of the tooth supports (8a, 8b and 9a, 9b, respectively) and at least one of the palate rests (6a, 6b and 7a, 7b, respectively) to which this tooth support (8a, 8b and 9a, 9b, respectively) is connected in order to thus fix the support surfaces (10) in their position against the palate (2).

2. Distractor according to claim 1, **characterized in that** palate rests (6a, 6b, 7a, 7b) and tooth supports (8a, 8b, 9a, 9b) of the respective anchoring part (4, 5) are directly connected to each other, more particularly in a rigid way.

3. Distractor according to claim 1 or 2, **characterized in that** the connection between the palate rests (6a, 6b, 7a, 7b) and tooth supports (8a, 8b, 9a, 9b) of the respective anchoring part (4, 5) is embodied as extending in such a way that it does not contact the palate (2).

4. Distractor according to one of claims 1 to 3, **characterized in that** the palate rest (6a, 6b, 7a, 7b) is embodied as an eyelet (16).

5. Distractor according to one of claims 1 to 4, **characterized in that** the anchoring part (4, 5) has a first and second arm (15a, 15b), which each connect a tooth support (8a, 8b, 9a, 9b) to a palate rest (6a, 6b, 7a, 7b), more particularly in a direct way.

6. Distractor according to claim 4 and 5, **characterized in that** the palate rest (6a, 6b, 7a, 7b) adjoins the first or second arm (15a, 15b, 15c) directly.

7. Distractor according to claim 5 or 6, **characterized in that** the tooth support (8a, 8b, 9a, 9b) has a third arm (15c), which connects the palate rests (6a, 6b, 7a, 7b) directly and is firmly connected to the respective expansion element (3a, 3b).

8. Distractor according to one of claims 1 to 4, **characterized in that** in each anchoring part (4, 5), the tooth supports (8a, 8b and 9a, 9b, respectively) are connected to the palate rests (6a, 6b and 7a, 7b, respectively) by means of a shared connector piece (55, 56).

9. Distractor according to claim 8, **characterized in that** the connector piece (55, 56) splits into two legs (55a, 55b and 56a, 56b, respectively), which each adjoin a palate rest (6a, 6b and 7a, 7b, respectively).

10. Distractor according to claim 8 or 9, **characterized in that** the legs (55a, 55b and 56a, 56b, respectively) extend in a V shape in relation to each other and/or the respective expansion element (3a, 3b) adjoins the connector piece (55, 56), which splits into the two legs (55a, 55b and 56a, 56b, respectively) in this region.

11. Distractor according to one of claims 1 to 10, **characterized in that** each anchoring part (4, 5) has two palate rests (6a, 6b, 7a, 7b) and/or two, three, or four tooth supports (8a, 8b, 9a, 9b).

12. Distractor according to one of claims 1 to 11, **characterized in that** the first tooth support (8a, 9a) has a molar band, which constitutes the first contact surface (13a).

13. Distractor according to one of claims 1 to 12, **characterized in that** the second tooth support (8b, 9b) has a half-lingual band that constitutes the first contact surface (13a).

14. Distractor according to one of claims 1 to 13, **characterized in that** the anchoring parts (4, 5) are embodied of one piece, more particularly embodied of one piece by means of an additive production method.

15. Distractor according to one of claims 1 to 14, **characterized in that** the through hole (11) has an end section (11b) tapering conically from the hole edge (11a).

16. Distractor according to one of claims 1 to 15, **characterized in that** the anchoring parts (4, 5) consists of a cobalt-chromium-tungsten alloy, more particular Co63,9Cr24,7W5,5Mo5.0Si.

17. Distractor according to one of claims 1 to 16, **characterized in that** the tooth supports (8a, 8b, 9a, 9b) of the two anchoring parts (4, 5) situated the farthest toward the outside relative to the longitudinal axis define a support plane (60) between themselves, within which the palate rests (6a, 6b, 7a, 7b) are positioned.

18. Distractor according to one of claims 1 to 17, **characterized in that** the first tooth support (8a, 9a) of each anchoring part (4, 5) is embodied so that it can be provided at a first tooth (12.1), which is a molar, and/or the second tooth support (8b, 9b) of each anchoring part (4, 5) is embodied so that it can be provided at a second tooth (12.2), which is a premolar.

19. Distractor according to one of claims 1 to 18, **characterized in that** the support surfaces (10) of the palate rests (6a, 6b, 7a, 7b) are each embodied so that they follow the contour of the palate (2) and/or the tooth supports (8a, 8b, 9a, 9b) are each embodied so that they follow the contour of the relevant tooth (12.1, 12.2).

20. Method for producing a distractor (1, 51) according to one of claims 1 to 19, **characterized in that** a digital image is taken of at least one section of a patient's jaw, the support surfaces (10) and tooth supports (8a, 8b, 9a, 9b) are adapted to the digital image and, with the aid of an additive production method, the anchoring parts (4, 5) are produced with the adapted support surfaces (10) and tooth supports (8a, 8b, 9a, 9b).

## Revendications

1. Distracteur destiné à une expansion palatine, avec une pièce d'expansion (3) qui présente au moins deux éléments d'expansion (3a, 3b) réglables à distance l'un de l'autre le long d'un axe longitudinal (L) et avec deux pièces d'ancrage (4, 5) qui sont fixées chacune à un élément d'expansion (3a, 3b), chaque pièce d'ancrage (4, 5) divisée en deux sections partielles (4a, 4b ou 5a, 5b) par l'axe longitudinal (L) présentant aussi bien sur chacune des deux sections partielles (4a, 4b ou 5a, 5b) au moins un appui palatin (6a, 6b, 7a, 7b) qu'au moins un support dentaire (8a, 8b, 9a, 9b), chaque appui palatin (6a, 6b, 7a, 7b) présentant une surface de support (10) pour s'appliquer contre le palais (2) et au moins un trou de passage (11) prévu dans la zone de la surface de support (10) pour un élément de fixation pour l'ancrage avec un os palatin du palais (2), chaque support dentaire (8a, 8b, 9a, 9b) présentant une première surface d'appui (13a) pour une surface latérale palatine d'une dent (12.1, 12.2) et une deuxième surface d'appui (13b) qui fait saillie de la première surface d'appui (13a) et forme un appui dentaire occlusal pour cette dent (12.1, 12.2), afin de positionner ainsi les surfaces de support (10) sur le palais (2), les pièces d'ancrage (4, 5) présentant chacune au moins deux supports dentaires (8a, 8b, 9a, 9b) et chaque section partielle (4a, 4b ou 5a, 5b) présentant au moins l'un des supports dentaires (8a, 8b ou 9a, 9b) et au moins l'un des appuis palatins (6a, 6b ou 7a, 7b) auquel ce support dentaire (8a, 8b ou 9a, 9b) est relié, afin de fixer ainsi les surfaces de support (10) dans leur position sur le palais (2).

2. Distracteur selon la revendication 1, **caractérisé en ce que** des appuis palatins (6a, 6b, 7a, 7b) et des supports dentaires (8a, 8b, 9a, 9b) de la pièce d'ancrage respective (4, 5) sont reliés directement entre eux, en particulier de manière rigide à la flexion.

3. Distracteur selon la revendication 1 ou 2, **caractérisé en ce que** la liaison entre les appuis palatins (6a, 6b, 7a, 7b) et les supports dentaires (8a, 8b, 9a, 9b) de la pièce d'ancrage respective (4, 5) est réalisée de manière à s'étendre sans contact par rapport au palais (2).

4. Distracteur selon l'une des revendications 1 à 3, **caractérisé en ce que** l'appui palatin (6a, 6b, 7a, 7b) est réalisé sous la forme d'un œillet (16).

5. Distracteur selon l'une des revendications 1 à 4, **caractérisé en ce que** la pièce d'ancrage (4, 5) présente un premier et un deuxième bras (15a, 15b) reliant chacun un support dentaire (8a, 8b, 9a, 9b) à un appui palatin (6a, 6b, 7a, 7b), notamment directement.

6. Distracteur selon les revendications 4 et 5, **caractérisé en ce que** l'appui palatin (6a, 6b, 7a, 7b) se raccorde directement au premier ou au deuxième bras (15a, 15b, 15c).

7. Distracteur selon la revendication 5 ou 6, **caractérisé en ce que** le support dentaire (8a, 8b, 9a, 9b) présente un troisième bras (15c) qui relie directement les appuis palatins (6a, 6b, 7a, 7b) et qui est solidaire de l'élément d'expansion respectif (3a, 3b).

8. Distracteur selon l'une des revendications 1 à 4, **caractérisé en ce que**, pour chaque pièce d'ancrage (4, 5), les supports dentaires (8a, 8b ou 9a, 9b) sont reliés aux appuis palatins (6a, 6b ou 7a, 7b) par une barrette commune (55, 56).

9. Distracteur selon la revendication 8, **caractérisé en ce que** la barrette (55, 56) se divise en deux branches (55a, 55b ou 56a, 56b) qui se raccordent chacune à un appui palatin (6a, 6b ou 7a, 7b).

10. Distracteur selon la revendication 8 ou 9, **caractérisé en ce que** les branches (55a, 55b ou 56a, 56b) s'étendent en forme de V l'une par rapport à l'autre et/ou **en ce que** l'élément d'expansion respectif (3a, 3b) se raccorde à la barrette (55, 56), qui se divise dans cette zone en les deux branches (55a, 55b ou 56a, 56b).

11. Distracteur selon l'une des revendications 1 à 10, **caractérisé en ce que** chaque pièce d'ancrage (4, 5) présente deux appuis palatins (6a, 6b, 7a, 7b) et/ou deux, trois ou quatre supports dentaires (8a, 8b, 9a, 9b).

12. Distracteur selon l'une des revendications 1 à 11, **caractérisé en ce que** le premier support dentaire (8a, 9a) présente une bande molaire qui forme la première surface d'appui (13a).

13. Distracteur selon l'une des revendications 1 à 12, **caractérisé en ce que** le deuxième support dentaire (8b, 9b) présente une demi-bande linguale qui forme la première surface d'appui (13a).

14. Distracteur selon l'une des revendications 1 à 13, **caractérisé en ce que** les pièces d'ancrage (4, 5) sont formées d'une seule pièce, en particulier d'une seule pièce par un procédé de fabrication additive.

15. Distracteur selon l'une des revendications 1 à 14, **caractérisé en ce que** le trou de passage (11) présente une section d'extrémité (11b) se rétrécissant de manière conique à partir du bord du trou (11a).

16. Distracteur selon l'une des revendications 1 à 15, **caractérisé en ce que** les pièces d'ancrage (4, 5) sont en alliage cobalt-chrome-tungstène, notamment Co63,9Cr24,7W5,5Mo5.0Si.

17. Distracteur selon l'une des revendications 1 à 16, **caractérisé en ce que** les supports dentaires (8a, 8b, 9a, 9b) des deux pièces d'ancrage (4, 5), situés le plus à l'extérieur par rapport à l'axe longitudinal, tendent entre eux une surface d'appui (60) à l'intérieur de laquelle sont disposés les appuis palatins (6a, 6b, 7a, 7b).

18. Distracteur selon l'une des revendications 1 à 17, **caractérisé en ce que** le premier support dentaire (8a, 9a) de chaque pièce d'ancrage (4, 5) est formé de manière prévisible sur une première dent (12.1) qui est une molaire arrière et/ou le deuxième support dentaire (8b, 9b) de chaque pièce d'ancrage (4, 5) est formé de manière prévisible sur une deuxième dent (12.2) qui est une molaire avant.

19. Distracteur selon l'une des revendications 1 à 18, **caractérisé en ce que** les surfaces d'appui (10) des appuis palatins (6a, 6b, 7a, 7b) sont réalisées chacune en suivant le contour du palais (2) et/ou les supports dentaires (8a, 8b, 9a, 9b) sont réalisés chacun en suivant le contour de la dent (12.1, 12.2) concernée.

20. Procédé de fabrication d'un distracteur (1, 51) selon l'une des revendications 1 à 19, **caractérisé en ce qu'**une image numérique d'au moins une partie d'une mâchoire d'un patient est saisie, les surfaces d'appui (10) et les supports dentaires (8a, 8b, 9a, 9b) sont adaptés à l'image numérique et les pièces d'ancrage (4, 5) avec les surfaces d'appui (10) et les supports dentaires (8a, 8b, 9a, 9b) adaptés sont fabriqués à l'aide d'un procédé de fabrication additive.
